# EUROPÄISCHE PATENTSCHRIFT

(11) **EP 3 811 915 B1**
(45) Veröffentlichungstag und Bekanntmachung des Hinweises auf die Patenterteilung: **15.11.2023**
(21) Anmeldenummer: 20202452.7
(22) Anmeldetag: 17.10.2020
(51) Int. Cl.: A61F 13/02, A61F 13/551, A61M 25/02

(54) **VERBUNDKÖRPER, VERWENDUNG EINES VERBUNDKÖRPERS UND SET MIT KOMPONENTEN EINER MEHRZAHL VON VERBUNDKÖRPERN**
COMPOSITE BODIES, USE OF A COMPOSITE BODY AND SET WITH COMPONENTS OF A PLURALITY OF COMPOSITE BODIES
CORPS COMPOSITE, UTILISATION D'UN CORPS COMPOSITE ET ENSEMBLE AVEC LES COMPOSANTS D'UNE PLURALITÉ DE CORPS COMPOSITES

(30) Priorität: 18.10.2019 DE 102019128226; 18.10.2019 DE 202019105809 U
(43) Veröffentlichungstag der Anmeldung: 28.04.2021
(73) Patentinhaber: L'Arcobaleno Claudia Chiello & Carsten Krahnert GbR, 78224 Singen (DE)
(72) Erfinder: Krahnert, Carsten, 88696 Owingen (DE); Chiello, Claudia, 78224 Singen (DE)
(74) Vertreter: RPK Patentanwälte Reinhardt und Kaufmann Partnerschaft mbB

(56) Entgegenhaltungen:
- EP-A1- 2 752 176
- WO-A1-2013/026879
- DE-T2- 69 232 777
- DE-T2- 69 409 102

## Beschreibung

### Stand der Technik

Die Erfindung betrifft einen Verbundkörper, eine Verwendung eines Verbundkörpers und ein Set mit Komponenten einer Mehrzahl von Verbundkörpern.

Es ist bekannt, temporär geschaffene nichtphysiologische Körperöffnungen, die insbesondere als Katheterzugänge dienen sollen, mit einer medizinischen Abdeckung zu versehen. Beispielsweise beschreibt die DE 2748882 A1 ein Pflaster zum Abdecken von intravenösen Kathetern, bei dem ein Katheter durch ein abdichtendes Polster hindurch geführt wird. Derartige Pflaster dienen dazu, über einen relativ kurzen Zeitraum die sichere und hygienische Platzierung des Katheters zu ermöglichen und etwaige Wundsekrete aufzunehmen.

EP 2752176 A1 offenbart eine Wundauflage zur Abdeckung von Kathetern. WO 2013/026879 A1 offenbart einen absorbierenden Hygiene- und Pflegeartikel. DE 69232777 T2 offenbart einen selbsthaftenden Verband und DE 69409102 T2 einen Wundverband mit einer absorbierenden Lage.

### Offenbarung der Erfindung

Die Aufgabe der Erfindung ist es, einen Verbundkörper zu schaffen, der für dauerhafte nichtphysiologische Körperöffnungen geeignet ist.

Eine weitere Aufgabe ist die Bereitstellung einer günstigen Darreichungsform derartiger Verbundkörper.

Eine weitere Aufgabe ist die Bereitstellung einer Verwendung eines Verbundkörpers.

Die Aufgaben werden durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Die in den Patentansprüchen einzeln aufgeführten Merkmale sind in technologisch sinnvoller Weise miteinander kombinierbar und können durch erläuternde Sachverhalte aus der Beschreibung und durch Details aus den Figuren ergänzt werden, wobei weitere Ausführungsvarianten der Erfindung aufgezeigt werden.

Es wird ein Verbundkörper vorgeschlagen, umfassend ein Kissen mit einer nichtklebenden Hülle, die mit einem hydrogelbildenden Absorbermaterial gefüllt ist, sowie ein selbstklebendes Befestigungselement, zur Anwendung bei der Abdeckung eines Katheterzugangs in einem Körper, der eine permanente Körperöffnung in Form eines Nabels aufweist, welcher den Katheterzugang bildet und der als Ausgang einer Neoblase ausgebildet ist, wobei das hydrogelbildende Absorbermaterial zur dauerhaften Aufnahme von aus der Körperöffnung austretendem Urin vorgesehen ist.

Das Kissen ist auswechselbar vorgesehen und das selbstklebende Befestigungselement mehrfach verwendbar. Dies ist kostengünstig, da das Kissen vergleichsweise preiswert im Vergleich zu dem Befestigungselement ist.

Der Verbundkörper dient zum Auflegen auf die permanente Körperöffnung, wobei das Kissen bestimmungsgemäß über der Körperöffnung anzuordnen ist. Die Hülle kann ein Vliesmaterial oder dergleichen sein. Ferner ist ein selbstklebendes Befestigungselement vorgesehen, das zur temporären Befestigung des Kissens im Bereich der Körperöffnung vorgesehen ist.

Im Gegensatz zu einem Wundpflaster, das dazu vorgesehen ist, nur relativ kurze Zeit über einer Wunde getragen zu werden, bis diese wieder verheilt ist, ist vorgesehen, dass die Verbundkörper praktisch permanent am Körper getragen werden können, was die Lebensqualität des Trägers verbessert.

Günstigerweise kann das hydrogelbildende Absorbermaterial einen sogenannten Superabsorber umfassen. Superabsorber sind an sich bekannt. Vorteilhaft werden Superabsorber eingesetzt, wie sie in Windeln, Binden, Tupfern oder dergleichen bekannt sind, die dazu vorgesehen sind, große Mengen an Flüssigkeit aufzunehmen. Superabsorber werden üblicherweise als Granulat angeboten und können ein Mehrfaches an Eigengewicht an Flüssigkeit unter entsprechender Volumenzunahme aufnehmen.

Übliche Superabsorber sind Kunststoffe, die als Granulat angeboten werden und die aus vernetzten, polaren Polymeren gebildet sind und daher die polaren Wassermoleküle elektrostatisch binden können. Durch die Wasseraufnahme bildet der Superabsorber ein Hydrogel. Häufig sind die Superabsorber-Partikel oberflächenbehandelt, indem an der Oberfläche der Partikel ein weiteres vernetztes Polymer angeordnet wird, welches das aufgequollene Hydrogel schützt und einen Wasser- bzw. Flüssigkeitsaustritt verhindert.

Der Verbundkörper ist besonders zur Anwendung und Pflege bei einer sogenannten Neoblase geeignet. Unter einer Neoblase versteht man in der Medizin, insbesondere der Urologie, einen aus Dünndarm erstellten kontinenten Harnblasenersatz.

Die nichtphysiologische Körperöffnung, welche den Ausgang der Neoblase bildet, ist bestimmungsgemäß permanent vorhanden.

Eine Neoblase kommt bei einer Zystektomie, d.h. bei einer Entfernung der Harnblase, zum Einsatz. Die Neoblase übernimmt die Reservoirfunktion der zuvor entfernten Harnblase. Ein Reservoir aus Darmschlingen wird mittels der sogenannten Detubularisierung konstruiert, wobei der Dünndarm längs aufgeschnitten und N-, S- oder W-förmig zu einer Darmplatte genäht wird. Deren Außenkanten werden miteinander verbunden. Das Ziel ist ein Reservoir, welches ohne großen Druckanstieg ein ausreichend großes Harnvolumen aufnehmen kann. Die Neoblase wird an Stelle der Harnblase in den Bauchraum eingesetzt und mit der Körperoberfläche (Haut) verbunden. Eine vorteilhafte Methode ist, den Ausgang der Neoblase zu verdrillen, um einen Verschluss der Neoblase zu erzeugen und den Ausgang mit dem Nabel zu verbinden. Betroffene Personen können sich selbst über den Nabel katheterisieren, um die Neoblase mehrmals am Tag, typischerweise sechsmal am Tag, zu entleeren.

Dies gewährleistet im Gegensatz zu den anderen Alternativen zur Harnableitung eine wesentlich bessere Lebensqualität und eine geringere Infektionsgefahr bei den betroffenen Personen.

Vorteilhaft erlaubt der erfindungsgemäße Verbundkörper die Aufnahme von Urinmengen, die trotz des Verschlusses der Neoblase in Form eines verdrillten Ausgangs austreten können, da der Verschluss nicht beliebig dicht sein kann, um die Einführung des Katheters zu ermöglichen. Beispielsweise kann beim Entfernen des Katheters oder bei Bewegungen, bei denen unkontrolliert Druck auf die Neoblase ausgeübt wird, eine Leckage auftreten. Vorteilhaft können auch Schleimmengen des Dünndarmgewebes im Urin aufgenommen werden. Eine Eigenschaft des die Neoblase bildenden Dünndarmgewebes ist, relativ große Mengen Schleim abzusondern, der sich dann im Urin wiederfindet. Der erfindungsgemäße Verbundkörper verhindert zuverlässig, dass sich an der Kleidung der betroffenen Personen unerwünschterweise feuchte Flecken bilden und vermeidet vorteilhaft eine unangenehme Geruchsbildung. Dies verbessert die Lebensqualität der betroffenen Personen weiter. Ebenso wird die Haut im Bereich der Körperöffnung vor Infektionen geschützt.

Das Kissen ist in der Lage, eine erhebliche Menge an Flüssigkeit aufzunehmen. Mit Vorteil wird zumindest das Kissen bei jeder Katheterisierung erneuert. Dies erlaubt eine relativ kleine Menge an Absorbermaterial, so dass der Verbundkörper mit dem Kissen unauffällig unter der Kleidung getragen werden kann.

Vorteilhaft ist das selbstklebende Befestigungselement biegeweich oder biegeschlaff ausgebildet, so dass sich das Befestigungselement, beispielsweise ein Pflaster, an das Kissen anpassen kann, wenn dieses Flüssigkeit aufnimmt und daraufhin aufquillt.

Vorteilhaft kann das selbstklebende Befestigungselement eine Abdeckung bilden, welche das Kissen vollständig überdeckt. Alternativ kann das Befestigungselement als umlaufender Rand ausgebildet sein und nur einen Randbereich des Kissens überdecken. Optional kann das selbstklebende Befestigungselement als umlaufender Rand am Kissen befestigt sein und bei Benutzung bestimmungsgemäß zwischen Kissen und Haut angeordnet sein.

Die Außenseite des selbstklebenden Befestigungselements kann durch ein Gewebe gebildet sein. Optional kann vorgesehen sein, dass das selbstklebende Befestigungselement eine glatte Oberfläche aufweist, so dass Kleidung leicht über das Befestigungselement gleiten kann.

Üblicherweise ist das Kissen preisgünstiger als das selbstklebende Befestigungselement, so dass es eine kostengünstige Lösung darstellt, wenn das selbstklebende Befestigungselement mehrfach verwendbar ist, insbesondere bei einem Dauergebrauch von Verbundkörpern.

Nach einer günstigen Ausgestaltung des Verbundkörpers kann das selbstklebende Befestigungselement einen hautverträglichen Kleber aufweisen. Die Haut um die Körperöffnung wird auch bei Daueranwendung wenig oder gar nicht durch den Kleber strapaziert. Der Kleber kann ganzflächig auf dem Befestigungselement vorgesehen sein oder nur in einem Randbereich.

Nach einer günstigen Ausgestaltung des Verbundkörpers kann das selbstklebende Befestigungselement einen hautverträglichen medizinischen Silikonkleber aufweisen. Derartige medizinische Silikonkleber sind für sensible Haut sehr geeignet und erlaubt ein sanftes Entfernen. Das Trägermaterial kann ein weicher Vliesstoff sein. Durch den medizinischen Silikonkleber lässt sich der Verbundkörper dementsprechend schmerzarm entfernen. Der Verbundkörper ist extrem hautschonend und kann einzeln steril verpackt sein.

Günstigerweise ist der Verbundkörper hypoallergen, atmungsaktiv, latexfrei ausgebildet. Der Kleber kann ganzflächig auf dem Befestigungselement vorgesehen sein oder nur in einem Randbereich. Der Randbereich liegt auf dem Körper auf, wenn der Verbundkörper mit dem Kissen über der Körperöffnung platziert ist.

Nach einer günstigen Ausgestaltung des Verbundkörpers kann das selbstklebende Befestigungselement als Pflaster ausgebildet sein, welches das Kissen abdeckt. Auf diese Weise kann der Verbundkörper bei der Anwendung aus zwei leicht verfügbaren Komponenten zusammengesetzt werden.

Nach einer günstigen Ausgestaltung des Verbundkörpers kann das selbstklebende Befestigungselement als freitragendes Element ausgebildet sein, das als umlaufender Rand an einem Randbereich des Kissens angeordnet ist. Dies spart Kosten und verringert den Materialverbrauch.

Das freitragende Befestigungselement kann mit seinem inneren Randbereich auf dem Kissen angeordnet sein, so dass der äußere Randbereich des Befestigungselements auf der Haut aufliegen kann. Alternativ kann das freitragende Befestigungselement als umlaufender Rand unter dem Kissen angeordnet sein und mit diesem verbunden sein. Alternativ kann eine Klebeschicht als Befestigungselement auf dem Kissen aufgetragen sein. Diese kann als umlaufender Rand ausgebildet sein oder mehrere Klebepunkte auf der Fläche des Kissens aufweisen. Vorteilhaft kann vermieden werden, dass Klebepunkte immer auf den gleichen Kontaktflächen auf der Haut aufgeklebt werden, was die Hautverträglichkeit des Verbundkörpers weiter verbessert.

Nach einer günstigen Ausgestaltung des Verbundkörpers kann das selbstklebende Befestigungselement als umlaufender Rand einstückig mit dem Kissen ausgebildet sein. Dies erlaubt eine besonders einfache Anwendung des Verbundkörpers.

Nach einer günstigen Ausgestaltung des Verbundkörpers kann das selbstklebende Befestigungselement mit seinem auf der Haut aufliegenden Klebebereich wenigstens 1 cm über Kanten des Kissens ragen. Vorteilhaft kann des Befestigungselement an einigen Stellen die Kante des Kissens deutlich weiter überragen. Damit kann sich der Verbundkörper auch bei Bewegungen des Körpers den Bewegungen anpassen. Der Tragekomfort ist erhöht.

Nach einer günstigen Ausgestaltung des Verbundkörpers kann das hydrogelbildende Absorbermaterial in unbenutztem Zustand ein Volumen von mindestens 12 cm³ aufweisen und vorteilhaft eine Dicke von höchstens 5 mm, günstigerweise höchstens 4 mm aufweisen. Das Kissen kann einen runden Querschnitt aufweisen. Vorteilhaft ist ein Durchmesser von etwa 5 - 6 cm. Alternativ kann das Kissen einen rechteckigen Querschnitt aufweisen. Optional kann es verschiedene Ausführungen des Verbundkörpers geben, bei denen ein Wechsel des Kissens in unterschiedlichen Zeiträumen erfolgen kann, wenn beispielsweise das Intervall zur Katheterisierung wegen besonderen Umständen, etwa wegen einer Reise, verlängert werden soll und das Kissen mehr Flüssigkeit aufnehmen muss, oder in der Nacht, um einen Wechsel des Kissens in der Nacht zu vermeiden.

Nach einer günstigen Ausgestaltung des Verbundkörpers kann das hydrogelbildende Absorbermaterial zur Aufnahme von wenigstens 12 cm³ Flüssigkeit wie Urin vorgesehen sein. Vorteilhaft kann damit ein Wechsel wenigstens des Kissens in ausreichend großen Zeiträumen erfolgen. Gegebenenfalls kann ein Kissen mit einem bestimmungsgemäßen Aufnahmevolumen von mehr als 12 cm³ vorgesehen sein, um unter besonderen Umständen längere Wechselintervalle zu ermöglichen.

Nach einem weiteren Aspekt der Erfindung wird ein Set mit einem Gehäuse und mit einer Mehrzahl von Komponenten von Verbundkörpern zur Anwendung bei der Abdeckung eines Katheterzugangs vorgeschlagen, wobei in dem Gehäuse eine erste Anzahl von Kissen mit Absorbermaterial und eine zweite Anzahl von selbstklebenden Rändern, insbesondere von Abdeckungen angeordnet ist und die erste Anzahl größer ist als die zweite Anzahl.

Dies erlaubt die Bereitstellung einer definierten Vorratsmenge an Verbundkörpern für Anwender, beispielsweise in Form von Monatspackungen. Die Komponenten können hygienisch einzeln verpackt sein und sind leicht zu entnehmen. Die Komponenten können übersichtlich in verschiedenen unterteilten Bereichen des Gehäuses angeordnet sein. Ein Anwender hat stets einen Überblick über die noch vorhandene Vorratsmenge.

Nach einer günstigen Ausgestaltung des Sets kann die erste Anzahl von Kissen dreimal größer sein als die zweite Anzahl von Befestigungselementen. Vorteilhaft kann eine Mehrfachverwendung von selbstklebenden Befestigungselementen erfolgen.

Nach einer günstigen Ausgestaltung kann das hydrogelbildende Absorbermaterial des Kissens zur Aufnahme von wenigstens 12 cm³ Urin vorgesehen sein. Gegebenenfalls kann ein Kissen mit einem bestimmungsgemäßen Aufnahmevolumen von mehr als 12 cm³ vorgesehen sein, um unter besonderen Umständen längere Wechselintervalle zu ermöglichen.

Günstigerweise kann das selbstklebende Befestigungselement einen hautverträglichen Kleber aufweisen. Die Haut um die Körperöffnung wird auch bei Daueranwendung wenig oder gar nicht durch den Kleber strapaziert. Der Kleber kann ganzflächig auf dem Befestigungselement vorgesehen sein oder nur in einem Randbereich.

Insbesondere kann das selbstklebende Befestigungselement einen hautverträglichen medizinischen Silikonkleber aufweisen. Derartige medizinische Silikonkleber sind für sensible Haut sehr geeignet und erlaubt ein sanftes Entfernen. Das Trägermaterial kann ein weicher Vliesstoff sein. Durch den medizinischen Silikonkleber lässt sich der Verbundkörper dementsprechend schmerzarm entfernen. Der Verbundkörper ist extrem hautschonend und kann einzeln steril verpackt sein. Günstigerweise ist der Verbundkörper hypoallergen, atmungsaktiv, latexfrei ausgebildet. Der Kleber kann ganzflächig auf dem Befestigungselement vorgesehen sein oder nur in einem Randbereich.

Nach einem weiteren Aspekt der Erfindung wird die Verwendung eines Verbundkörpers vorgeschlagen, umfassend ein Kissen mit einer nichtklebenden Hülle, die mit einem hydrogelbildenden Absorbermaterial gefüllt ist, sowie ein selbstklebendes Befestigungselement, zur Abdeckung einer permanenten Körperöffnung in Form eines Nabels, der einen Katheterzugang gebildet, welcher als Ausgang einer Neoblase ausgebildet ist, wobei das hydrogelbildende Absorbermaterial zur dauerhaften Aufnahme von aus der Körperöffnung austretendem Urin vorgesehen ist.

Günstigerweise kann das hydrogelbildende Absorbermaterial zur dauerhaften Aufnahme von wenigstens 12 cm³ von aus der Körperöffnung austretendem Urin vorgesehen sein. Vorteilhaft kann damit ein Wechsel wenigstens des Kissens in ausreichend großen Zeiträumen erfolgen. Gegebenenfalls kann ein Kissen mit einem bestimmungsgemäßen Aufnahmevolumen von mehr als 12 cm³ vorgesehen sein, um unter besonderen Umständen längere Wechselintervalle zu ermöglichen.

Das Kissen ist auswechselbar vorgesehen, und das selbstklebende Befestigungselement ist mehrfach verwendbar. Dies ist kostengünstig, da das Kissen vergleichsweise preiswert im Vergleich zu dem Befestigungselement ist.

Dabei kann das selbstklebende Befestigungselement einen hautverträglichen Kleber aufweisen. Insbesondere kann das selbstklebende Befestigungselement einen hautverträglichen medizinischen Silikonkleber aufweisen.

Der Kleber kann ganzflächig auf dem Befestigungselement vorgesehen sein oder nur in einem Randbereich. Der Randbereich wird auf den Körper aufgelegt, wenn der Verbundkörper mit dem Kissen über der Körperöffnung platziert wird.

Das selbstklebende Befestigungselement kann als Pflaster ausgebildet sein, welches das Kissen abdeckt. Alternativ kann das selbstklebende Befestigungselement als freitragendes Element ausgebildet sein, das als umlaufender Rand an einem Randbereich des Kissens angeordnet wird.

Das selbstklebende Befestigungselement kann als umlaufender Rand einstückig mit dem Kissen ausgebildet sein.

Das selbstklebende Befestigungselement kann mit seinem auf der Haut aufliegenden Klebebereich wenigstens 1 cm über Kanten des Kissens ragen.

Das hydrogelbildende Absorbermaterial kann in unbenutztem Zustand ein Volumen von mindestens 12 cm³ aufweisen und vorteilhaft eine Dicke von höchstens 5 mm, günstigerweise höchstens 4 mm aufweisen.

### Zeichnung

Weitere Vorteile ergeben sich aus der folgenden Zeichnungsbeschreibung. In den Zeichnungen sind Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen beispielhaft:
- Fig. 1: in Draufsicht schematisch einen Verbundkörper mit einem selbstklebenden Befestigungselement und einem Kissen nach einem Ausführungsbeispiel der Erfindung;
- Fig. 2: einen Längsschnitt durch eine schematisch dargestellte Anwendung des Verbundkörpers nach Figur 1 an einer nichtphysiologischen Körperöffnung am Ausgang einer Neoblase;
- Fig. 3: einen Längsschnitt durch einen schematisch dargestellten Verbundkörper nach einem Ausführungsbeispiel der Erfindung;
- Fig. 4: einen Längsschnitt durch einen schematisch dargestellten Verbundkörper nach einem Ausführungsbeispiel der Erfindung;
- Fig. 5: in Draufsicht ein Set für einen Verbundkörper mit selbstklebendem Befestigungselement und Kissen nach einem Ausführungsbeispiel der Erfindung;
- Fig. 6: in Draufsicht ein selbstklebendes Befestigungselement nach einem Ausführungsbeispiel der Erfindung;
- Fig. 7: in Draufsicht ein Kissen nach einem Ausführungsbeispiel der Erfindung.

### Ausführungsformen der Erfindung

In den Figuren sind gleichartige oder gleichwirkende Komponenten mit gleichen Bezugszeichen beziffert. Die Figuren zeigen lediglich Beispiele und sind nicht beschränkend zu verstehen.

Bevor die Erfindung im Detail beschrieben wird, ist darauf hinzuweisen, dass sie nicht auf die jeweiligen Bauteile der Vorrichtung sowie die jeweiligen Verfahrensschritte beschränkt ist, da diese Bauteile und Verfahren variieren können. Die hier verwendeten Begriffe sind lediglich dafür bestimmt, besondere Ausführungsformen zu beschreiben und werden nicht einschränkend verwendet. Wenn zudem in der Beschreibung oder in den Ansprüchen die Einzahl oder unbestimmte Artikel verwendet werden, bezieht sich dies auch auf die Mehrzahl dieser Elemente, solange nicht der Gesamtzusammenhang eindeutig etwas Anderes deutlich macht.

Im Folgenden verwendete Richtungsterminologie mit Begriffen wie "links", "rechts", "oben", "unten", "davor" "dahinter", "danach" und dergleichen dient lediglich dem besseren Verständnis der Figuren und soll in keinem Fall eine Beschränkung der Allgemeinheit darstellen. Die dargestellten Komponenten und Elemente, deren Auslegung und Verwendung können im Sinne der Überlegungen eines Fachmanns variieren und an die jeweiligen Anwendungen angepasst werden.

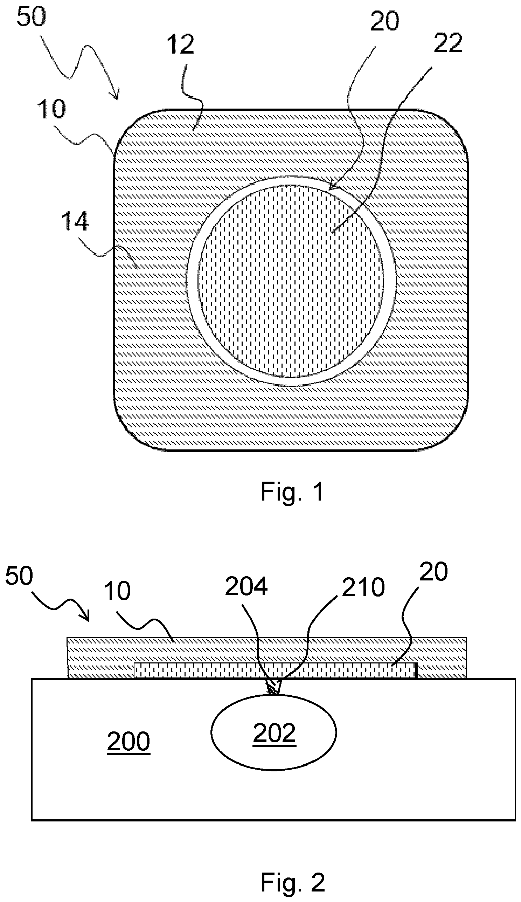

Figur 1 zeigt in Draufsicht einen Verbundkörper 50 mit einem selbstklebenden Befestigungselement 10 und einem Kissen 20 nach einem Ausführungsbeispiel der Erfindung, wie er zur Anwendung bei der Abdeckung eines Katheterzugangs vorgesehen ist, um eine nichtphysiologische Körperöffnung 210 abzudecken, welche den Katheterzugang bildet. Figur 2 zeigt einen Längsschnitt durch eine schematisch dargestellte Anwendung des Verbundkörpers 50 nach Figur 1 an einer Neoblase 202 in einem Körper 200, wobei ein zumindest temporär verschlossener Ausgang 204 der Neoblase 202 mit der Körperöffnung 210, beispielsweise einem Nabel, verbunden ist und über diese Körperöffnung 210 für einen Katheter zugänglich ist, um die Neoblase 202 zu entleeren. Der Nabel bildet dann eine permanente nichtphysiologische Körperöffnung 210.

Der Verbundkörper 50 ist zum Auflegen auf die permanente Körperöffnung 210 vorgesehen, welche den Ausgang 204 der Neoblase 202 bildet. Das Kissen 20, das über der Körperöffnung 210 anzuordnen ist, weist eine nichtklebende Hülle 22 auf, die mit einem hydrogelbildenden Absorbermaterial 24 zur dauerhaften Aufnahme von Urin, welche durch unvermeidbare Leckagen aus der Neoblase 202 austritt, gefüllt ist. Das Kissen 20 ist durch das selbstklebende Befestigungselement 10 am Körper 200 befestigt.

Im Gegensatz zu einem Wundpflaster, das nur relativ kurze Zeit über einer Wunde getragen wird, bis diese wieder verheilt ist, ist vorgesehen, dass die Verbundkörper 50 praktisch permanent am Körper 200 getragen werden können, was die Lebensqualität des Trägers verbessert.

Das hydrogelbildende Absorbermaterial 24 kann einen sogenannten Superabsorber umfassen. Superabsorber sind dazu vorgesehen, große Mengen an Flüssigkeit aufzunehmen. Superabsorber werden üblicherweise als Granulat angeboten und können ein Mehrfaches an Eigengewicht an Flüssigkeit unter entsprechender Volumenzunahme aufnehmen. Übliche Superabsorber sind Kunststoffe, die als Granulat angeboten werden und die aus vernetzten, polaren Polymeren gebildet sind und daher die polaren Wassermoleküle elektrostatisch binden können. Durch die Wasseraufnahme bildet der Superabsorber ein Hydrogel. Häufig sind die Superabsorber-Partikel oberflächenbehandelt, indem an der Oberfläche der Partikel ein weiteres vernetztes Polymer angeordnet wird, welches das aufgequollene Hydrogel schützt und einen Wasser- bzw. Flüssigkeitsaustritt verhindert. Hydrogele können alle vernetzten Polymere bilden, die polar sind (z. B. Polyacrylamid, Polyvinylpyrrolidon, Amylopektin, Gelatine, Cellulose). Häufig wird ein Copolymer aus Acrylsäure (Propensäure) oder Natriumacrylat, einem Natriumsalz der Acrylsäure einerseits und Acrylamid andererseits verwendet.

In dem gezeigten Ausführungsbeispiel weist das Kissen 20 einen runden Querschnitt auf. Alternativ kann auch ein rechteckiger Querschnitt des Kissens 20 vorgesehen sein. Das Befestigungselement 10 kann, wie dargestellt, einen rechteckigen Querschnitt aufweisen, oder auch einen runden Querschnitt.

Auf der der Haut zugewandten Seite ist das Befestigungselement 10 mit einer Schicht eines Klebers 12 beschichtet, der vorzugsweise ein hautverträglicher medizinischer Silikonkleber ist. Günstigerweise kann das Befestigungselement 10 mehrfach verwendet werden, beispielsweise dreimal. Dann kann das Kissen 20 ausgetauscht werden, wenn der Anwender die Neoblase 202 mit einem Katheter entleert und ein frisches Kissen 20 mit demselben Befestigungselement 10 wieder über der Körperöffnung 210 angebracht wird. Das Befestigungselement 10 ist verglichen mit dem Kissen 20 relativ teuer.

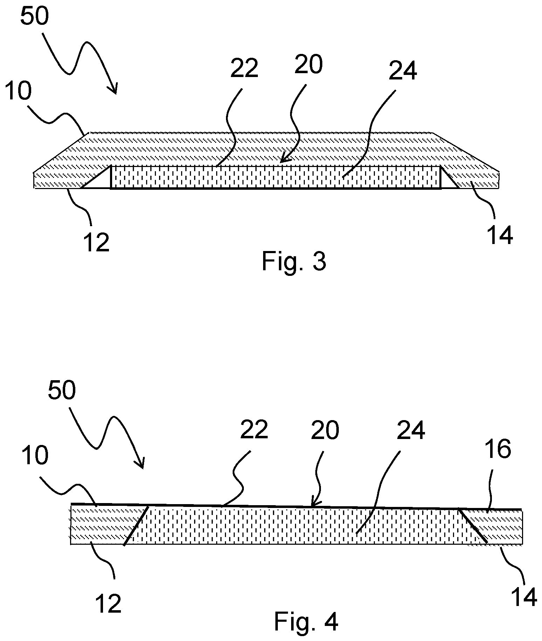

Figur 3 zeigt einen Längsschnitt durch einen schematisch dargestellten Verbundkörper 50 nach einem Ausführungsbeispiel der Erfindung. Das Befestigungselement 10 ist in Form eines Pflasters ausgebildet. Das Befestigungselement 10 überdeckt das Kissen und überragt dessen Kanten auf allen Seiten mit einem Kleberand 14. Der Kleber 12 kann ganzflächig vorgesehen sein oder nur im Bereich des Kleberands 14.

Figur 4 zeigt einen Längsschnitt durch einen schematisch dargestellten Verbundkörper 50 nach einem weiteren Ausführungsbeispiel der Erfindung. Das Befestigungselement 10 ist in Form eines freitragenden umlaufenden Randelements 16 ausgebildet. Das Befestigungselement 10 überdeckt das Kissen und überragt dessen Kanten auf allen Seiten mit einem Kleberand 14. Der Kleber 12 ist im Bereich des Randelements 16 vorzugsweise ganzflächig auf dem Kleberand 14 vorgesehen.

Das Befestigungselement 10 ist vorzugsweise sehr dünn ausgeführt und insbesondere dünner als das Kissen 20. Die Schicht des Klebers 12 kann in einer nicht dargestellten Ausgestaltung auch direkt auf dem Kissen 20 aufgebracht sein.

In einer weiteren nicht dargestellten Ausgestaltung kann das Befestigungselement 10 als umlaufendes Randelement auch unter dem Kissen 20 angeordnet sein.

Dann kann sich das Befestigungselement 10 weiter als die Kanten des Kissens 20 auf der Haut erstrecken, oder das Kissen 20 überdeckt das Befestigungselement völlig oder weitgehend.

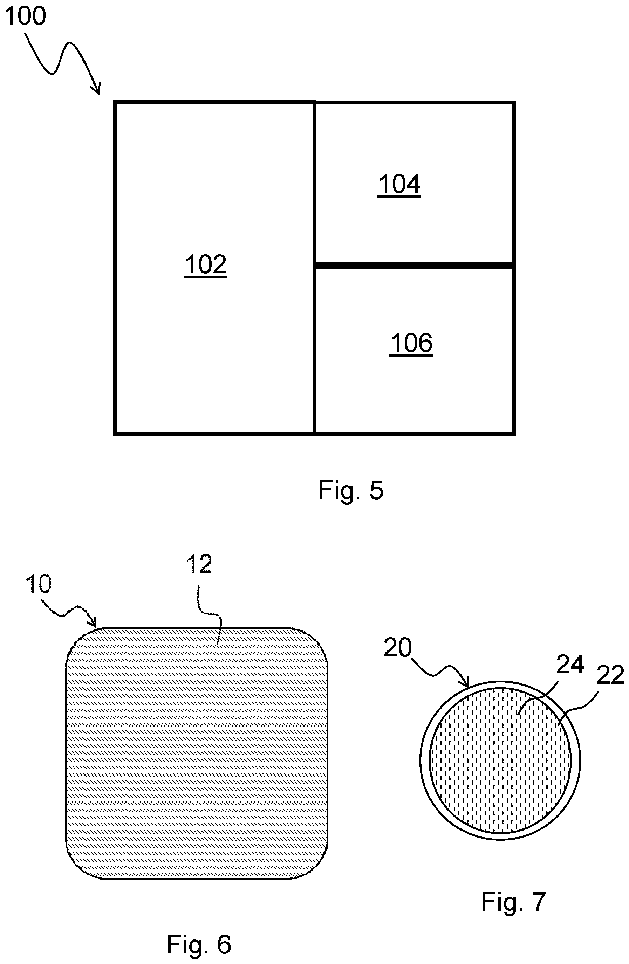

Figur 5 zeigt in Draufsicht ein Set für einen Verbundkörper 50 mit selbstklebendem Befestigungselement 10 und Kissen 20 nach einem Ausführungsbeispiel der Erfindung. Figur 6 zeigt in Draufsicht ein selbstklebendes Befestigungselement 10 in Form eines Pflasters für das Set und Figur 7 zeigt in Draufsicht ein Kissen 20 für das Set.

Das Set ist vorzugsweise eine Vorratspackung für Komponenten der Verbundkörper 50 und umfasst ein Gehäuse 100, in dem eine erste Anzahl von selbstklebenden Befestigungselementen10 in einem Fach 102 angeordnet ist und eine zweite Anzahl von nichtklebenden Kissen 20, die mit Absorbermaterial 24 gefüllt sind. Da das Befestigungselement 10 wiederverwendbar ist, ist die erste Anzahl kleiner als die zweite Anzahl, insbesondere sind dreimal mehr Kissen 20 vorhanden als Befestigungselemente 10, wenn die Befestigungselemente 10 dreimal verwendbar sind.

Das Kissen wird einfach auf dem Körper 200 (Figur 2) über der Körperöffnung 210 platziert, das Befestigungselement 10 darüber gelegt und so an Ort und Stelle fixiert, bis das Kissen 20 gewechselt wird. Zweckmäßigerweise erfolgt dies, wenn die Neoblase 202 (Figur 2) mit einem Katheter entleert wird.

## Patentansprüche

1. Verbundkörper (50), umfassend ein Kissen (20) mit einer nichtklebenden Hülle (22), die mit einem hydrogelbildenden Absorbermaterial (24) gefüllt ist, sowie ein selbstklebendes Befestigungselement (10), zur Anwendung bei der Abdeckung eines Katheterzugangs in einem Körper (200), der eine permanente Körperöffnung (210) in Form eines Nabels aufweist, welcher den Katheterzugang bildet und der als Ausgang (204) einer Neoblase (202) ausgebildet ist,
**dadurch gekennzeichnet, dass**
das hydrogelbildende Absorbermaterial (24) zur dauerhaften Aufnahme von aus der Körperöffnung (210) austretendem Urin vorgesehen ist, wobei das Kissen (20) auswechselbar vorgesehen ist und das selbstklebende Befestigungselement (10) mehrfach verwendbar ist.

2. Verbundkörper zur Anwendung bei der Abdeckung eines Katheterzugangs nach Anspruch 1, **dadurch gekennzeichnet, dass** das selbstklebende Befestigungselement (10) einen hautverträglichen Kleber (12) aufweist, insbesondere dass das selbstklebende Befestigungselement (10) als Kleber (12) einen hautverträglichen medizinischen Silikonkleber aufweist.

3. Verbundkörper zur Anwendung bei der Abdeckung eines Katheterzugangs nach einem der vorhergehenden Ansprüche, **dadurch gekennzeichnet, dass** das selbstklebende Befestigungselement (10) als Pflaster ausgebildet ist, welches das Kissen (20) abdeckt.

4. Verbundkörper zur Anwendung bei der Abdeckung eines Katheterzugangs nach einem der vorhergehenden Ansprüche, **dadurch gekennzeichnet, dass** das selbstklebende Befestigungselement (10) als freitragendes Element ausgebildet ist, das als umlaufender Rand (14) an einem Randbereich des Kissens (20) angeordnet ist.

5. Verbundkörper zur Anwendung bei der Abdeckung eines Katheterzugangs nach einem der Ansprüche 1 bis 3, **dadurch gekennzeichnet, dass** das selbstklebende Befestigungselement (10) als umlaufender Rand (14) einstückig mit dem Kissen (20) ausgebildet ist.

6. Verbundkörper zur Anwendung bei der Abdeckung eines Katheterzugangs nach einem der vorhergehenden Ansprüche, **dadurch gekennzeichnet, dass** das selbstklebende Befestigungselement (10) mit seinem auf der Haut aufliegenden Klebebereich (16) wenigstens 1 cm über Kanten des Kissens (20) ragt.

7. Verbundkörper zur Anwendung bei der Abdeckung eines Katheterzugangs nach einem der vorhergehenden Ansprüche, **dadurch gekennzeichnet, dass** das hydrogelbildende Absorbermaterial (24) in unbenutztem Zustand ein Volumen von mindestens 12 cm³ aufweist.

8. Verbundkörper zur Anwendung bei der Abdeckung eines Katheterzugangs nach einem der vorhergehenden Ansprüche, **dadurch gekennzeichnet, dass** das hydrogelbildende Absorbermaterial (24) zur Aufnahme von wenigstens 12 cm³ Flüssigkeit vorgesehen ist.

9. Set mit einem Gehäuse (100) und mit einer Mehrzahl von Komponenten (10, 20) von Verbundkörpern (50) zur Anwendung bei der Abdeckung eines Katheterzugangs nach einem der vorhergehenden Ansprüche, wobei in dem Gehäuse (100) eine erste Anzahl von Kissen (20) mit Absorbermaterial (24), und eine zweite Anzahl von selbstklebenden Befestigungselementen (10) angeordnet ist und die erste Anzahl größer ist als die zweite Anzahl.

10. Set nach Anspruch 9, **dadurch gekennzeichnet, dass** die erste Anzahl von Kissen (20) dreimal größer ist als die zweite Anzahl von Befestigungselementen (10).

11. Set nach Anspruch 9 oder 10, **dadurch gekennzeichnet, dass** das Kissen (20) zur Aufnahme von wenigstens 12 cm³ Urin vorgesehen ist.

12. Verwendung eines Verbundkörpers (50), umfassend ein Kissen (20) mit einer nichtklebenden Hülle (22), die mit einem hydrogelbildenden Absorbermaterial (24) gefüllt ist, sowie ein selbstklebendes Befestigungselement (10), zur Abdeckung einer permanenten Körperöffnung (210) in Form eines Nabels, der einen Katheterzugang bildet, welcher als Ausgang (204) einer Neoblase (202) ausgebildet ist,
wobei das hydrogelbildende Absorbermaterial (24) zur dauerhaften Aufnahme von aus der Körperöffnung (210) austretendem Urin vorgesehen ist.

13. Verwendung nach Anspruch 12, wobei das hydrogelbildende Absorbermaterial (24) zur dauerhaften Aufnahme von wenigstens 12 cm³ von aus der Körperöffnung (210) austretendem Urin vorgesehen ist.

## Claims

1. Composite body (50), comprising a pad (20) with a non-adhesive cover (22) filled with a hydrogel-forming absorbent material (24), and a self-adhesive fastening element (10) for use in covering a catheter port in a body (200) which has a permanent body orifice (210) in the form of a navel which forms the catheter port and which is formed as an output (204) of a neobladder (202),
**characterised in that**
the hydrogel-forming absorbent material (24) is provided for permanent absorption of urine leaking from the body orifice (210), wherein
the pad (20) is provided to be replaceable and the self-adhesive fastening element (10) can be used multiple times.

2. Composite body for use in covering a catheter port according to claim 1, **characterised in that** the self-adhesive fastening element (10) has a skin-compatible adhesive (12), in particular **in that** the self-adhesive fastening element (10) has a skin-compatible medical silicone adhesive as the adhesive (12).

3. Composite body for use in covering a catheter port according to any one of the preceding claims, **characterised in that** the self-adhesive fastening element (10) is formed as a plaster which covers the pad (20).

4. Composite body for use in covering a catheter port according to any one of the preceding claims, **characterised in that** the self-adhesive fastening element (10) is formed as a self-supporting element which is arranged on an edge region of the pad (20) as a peripheral edge (14).

5. Composite body for use in covering a catheter port according to any one of claims 1 to 3, **characterised in that** the self-adhesive fastening element (10) is formed in one piece with the pad (20) as a peripheral edge (14).

6. Composite body for use in covering a catheter port according to any one of the preceding claims, **characterised in that** the self-adhesive fastening element (10) with its adhesive region (16) lying on the skin projects at least 1 cm over the edges of the pad (20).

7. Composite body for use in covering a catheter port according to any one of the preceding claims, **characterised in that** the hydrogel-forming absorbent material (24) has a volume of at least 12 cm³ when unused.

8. Composite body for use in covering a catheter port according to any one of the preceding claims, **characterised in that** the hydrogel-forming absorbent material (24) is provided for absorbing at least 12 cm³ fluid.

9. Kit having a housing (100) and a plurality of components (10, 20) of composite bodies (50) for use in covering a catheter port according to any one of the preceding claims, wherein a first number of pads (20) having absorbent material (24) and a second number of self-adhesive fastening elements (10) are arranged in the housing (100) and the first number is greater than the second number.

10. Kit according to claim 9, **characterised in that** the first number of pads (20) is three times greater than the second number of fastening elements (10).

11. Kit according to claim 9 or 10, **characterised in that** the pad (20) is provided for absorbing at least 12 cm³ urine.

12. Use of a composite body (50), comprising a pad (20) with a non-adhesive cover (22) which is filled with a hydrogel-forming absorbent material (24) and a self-adhesive fastening element (10) for covering a permanent body orifice (210) in the form of a navel which forms a catheter port which is formed as an output (204) of a neobladder (202),
wherein the hydrogel-forming absorbent material (24) is provided for permanent absorption of urine leaking from the body orifice (210).

13. Use according to claim 12, wherein the hydrogel-forming absorbent material (24) is provided for permanent absorption of at least 12 cm³ urine leaking from the body orifice (210).

## Revendications

1. Corps composite (50) comprenant un patch (20) avec une enveloppe non adhésive (22) remplie d'un matériau absorbant formant un hydrogel (24) ainsi qu'un élément de fixation auto-adhésif (10), destiné à être utilisé pour couvrir un accès de cathéter dans un corps (200) qui présente une ouverture corporelle (210) permanente sous la forme d'un ombilic, lequel forme l'accès de cathéter et qui est conçu comme sortie (204) d'une néo-vessie (202),
**caractérisé en ce que**
le matériau absorbant (24) formant un hydrogel est prévu pour absorber de manière permanente l'urine s'échappant de l'ouverture corporelle (210), dans lequel
le patch (20) est prévu pour pouvoir être remplacé et l'élément de fixation auto-adhésif (10) peut être utilisé plusieurs fois.

2. Corps composite destiné à être utilisé pour recouvrir un accès à un cathéter selon la revendication 1, **caractérisé en ce que** l'élément de fixation auto-adhésif (10) présente une colle (12) tolérée par la peau, en particulier **en ce que** l'élément de fixation auto-adhésif (10) présente comme colle (12) une colle silicone médicale tolérée par la peau.

3. Corps composite destiné à être utilisé pour recouvrir un accès de cathéter selon l'une quelconque des revendications précédentes, **caractérisé en ce que** l'élément de fixation auto-adhésif (10) est conçu comme un pansement qui recouvre le patch (20).

4. Corps composite destiné à être utilisé pour recouvrir un accès de cathéter selon l'une quelconque des revendications précédentes, **caractérisé en ce que** l'élément de fixation auto-adhésif (10) est réalisé sous la forme d'un élément autoportant qui est agencé sous la forme d'un bord périphérique (14) sur une zone de bord du patch (20).

5. Corps composite destiné à être utilisé pour recouvrir un accès de cathéter selon l'une quelconque des revendications 1 à 3, **caractérisé en ce que** l'élément de fixation auto-adhésif (10) est réalisé d'un seul tenant avec le patch (20) sous la forme d'un bord périphérique (14).

6. Corps composite destiné à être utilisé pour recouvrir un accès de cathéter selon l'une quelconque des revendications précédentes, **caractérisé en ce que** l'élément de fixation auto-adhésif (10) dépasse d'au moins 1 cm des bords du patch (20) par sa zone adhésive (16) reposant sur la peau.

7. Corps composite destiné à être utilisé pour recouvrir un accès de cathéter selon l'une quelconque des revendications précédentes, **caractérisé en ce que** le matériau absorbant (24) formant un hydrogel présente un volume d'au moins 12 cm³ à l'état non utilisé.

8. Corps composite destiné à être utilisé pour couvrir un accès de cathéter selon l'une quelconque des revendications précédentes, **caractérisé en ce que** le matériau absorbant formant un hydrogel (24) est prévu pour contenir au moins 12 cm³ de liquide.

9. Ensemble avec un boîtier (100) et avec une pluralité de composants (10, 20) de corps composites (50) destiné à être utilisé dans le recouvrement d'un accès de cathéter selon l'une quelconque des revendications précédentes, dans lequel un premier nombre de patchs (20) avec un matériau absorbant (24), et un second nombre d'éléments de fixation auto-adhésifs (10) sont agencés dans le boîtier (100), et le premier nombre est plus grand que le second nombre.

10. Ensemble selon la revendication 9, **caractérisé en ce que** le premier nombre de patchs (20) est trois fois plus grand que le second nombre d'éléments de fixation (10).

11. Ensemble selon la revendication 9 ou 10, **caractérisé en ce que** le patch (20) est prévu pour recevoir au moins 12 cm³ d'urine.

12. Utilisation d'un corps composite (50) comprenant un patch (20) avec une enveloppe non adhésive (22) remplie d'un matériau absorbant formant un hydrogel (24), ainsi qu'un élément de fixation auto-adhésif (10), pour recouvrir une ouverture corporelle permanente (210) sous la forme d'un ombilic, qui forme un accès de cathéter, lequel est conçu comme sortie (204) d'une néo-vessie (202),
dans lequel le matériau absorbant (24) formant un hydrogel est prévu pour absorber de manière permanente l'urine s'échappant de l'ouverture corporelle (210).

13. Utilisation selon la revendication 12, dans laquelle le matériau absorbant formant un hydrogel (24) est prévu pour absorber de manière permanente au moins 12 cm³ d'urine s'échappant de l'ouverture corporelle (210).
